(19) Europäisches Patentamt
European Patent Office
Office européen des brevets

(11) EP 4 186 886 A1

(12) EUROPEAN PATENT APPLICATION

(43) Date of publication:
**31.05.2023 Bulletin 2023/22**

(21) Application number: **21211470.6**

(22) Date of filing: **30.11.2021**

(51) International Patent Classification (IPC):
***C07C 67/00*** *(2006.01)* ***C07C 67/31*** *(2006.01)*
***C07C 69/86*** *(2006.01)* ***C07C 69/94*** *(2006.01)*
***C07D 311/80*** *(2006.01)* ***C07D 493/04*** *(2006.01)*

(52) Cooperative Patent Classification (CPC):
(C-Sets available)
**C07C 69/86; C07C 67/00; C07C 67/31; C07D 311/80; C07D 493/04** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Tresco Labs GmbH**
**65929 Frankfurt am Main (DE)**

(72) Inventor: **COSKY, Eric S.**
**65929 Frankfurt am Main (DE)**

(74) Representative: **Fuchs Patentanwälte Partnerschaft mbB**
**Tower 185**
**Friedrich-Ebert-Anlage 35-37**
**60327 Frankfurt am Main (DE)**

# (54) PROCESS FOR THE SYNTHESIS AND PURIFICATION OF CANNABINOIC ACIDS AND ACYLATED DERIVATIVES THEREOF

(57) The invention relates to a novel process for the synthesis and/or purification of tetrahydrocannabinolic acid (THCA) alkanoates and/or cannabidiolic acid (CBDA) di- alkanoates. The invention also provides novel derivatives of CBDA di-alkanoates and THCA alkanoates according to Formula I, wherein $R^1$ = methyl-4-(prop-1-en-2-yl)-cyclohex-1-ene-3-yl (limonenyl); wherein $R^2$ = acetate, propionate, butyrate, or OH; wherein $R^3$ = propyl, pentyl, or heptyl; wherein $R^4$ = methyl, ethyl, or propyl; wherein $R^5$ = methyl, ethyl, or propyl, or wherein $R^1$ = methyl-4-(2-propyl)-cyclohex-1-ene-3-yl or methyl-4-(2-propyl)-cyclohex-5-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ = methyl, ethyl, or propyl, and wherein $R^5$ = methyl, ethyl, or propyl, or wherein $R^1$ = methyl-4-(2-propyl)-cyclohex-1-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ and $R^5$ vanish, i.e. are radicals forming a direct bond to each other.
(Formula I)

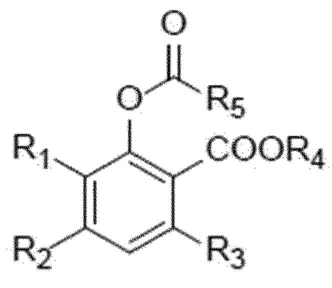

I

EP 4 186 886 A1

(52) Cooperative Patent Classification (CPC): (Cont.)

C-Sets
**C07C 67/00, C07C 69/94;**
**C07C 67/31, C07C 69/86**

## Description

The invention relates to a novel process for the synthesis and/or purification of tetrahydrocannabinolic acid (THCA) alkanoates and/or cannabidiolic acid (CBDA) di- alkanoates. The invention also provides novel derivatives of CBDA di-alkanoates and THCA alkanoates.

## Background

Cannabinoids are terpene phenols found in flowering *Cannabinaceae* plants of the genus *Cannabis,* namely in the species *Cannabis sativa, Cannabis indica,* and *Cannabis ruderalis* and are subdivided into 12 classes: cannabigerols (CBG), cannabichromenes (CBC), cannabidiols (CBD), cannabinodiols (CBND), tetrahydrocannabinols (THC), cannabinols (CBN), cannabitriols (CBT), cannabielsoins (CBE), isocannabinoids, cannabicyclols (CBL), cannabicitrans (CBT) and cannabichromanones (CBCN).

Cannabis extracts and concentrates (oils and resins) containing different amounts of different classes of cannabinoids depending on the extraction process have found wide applications as food additives and cosmetics. Some cannabinoids like, for instance, (-)-*trans*-cannabidiol (CBD) and (-)-$\Delta^9$-*trans*-tetrahydrocannabinol ($\Delta^9$-THC, Dronabinol) show distinct physiological effects by binding to the cannabinoid receptors CB1 and CB2 of the endocannabinoid system, and are also used as pharmaceuticals modulating appetite, pain, mood and memory (CB1) or influencing the immune system (CB2). The biosynthesis of the latter cannabinoids comprises the formation of cannabidiolic acid (CBDA) which is enzymatically decarboxylated to CBD or cyclized to tetrahydrocannabinolic acid (THCA). Decarboxylation of CBDA and THCA to CBD and THC, respectively also occurs chemically at elevated temperatures during the extraction process.

Methods for the isolation of cannabinoids are known in the art.

Formato et al. (Molecules 25, 2020, 2638) reviewed aspects on (-)-cannabidiolic acid. CBDA is bioactive and acts as an anti-inflammatory, anti-emetic, anti-convulsant and anti-carcinogenic drug. Formato et al. describe a procedure for extracting CBDA together with THCA from the "pollen", or trichomes, of industrial hemp cultivars and managed to identify analytical quantities of purified CBDA using preparative thin-layer chromatography by which they distinguished CBDA from THCA. However, a solitary spot on a glass slide sprayed with silica, is not a convenient vehicle for arriving at even research quantities of an organic compound.

Current synthetic routes to CBDA, including CBDA purification from Hemp biomass, are still cumbersome and do not provide the desired purities.

Current methodologies to isolate and purify cannabinoids suffer to a large extent from the known fact that, with the exception of CBD, pure cannabinoids are typically oily compounds that are difficult, if not impossible, to crystallize. Additionally, valuable intermediates during synthesis and available through bio-production, such as THCA and CBDA, tend to decarboxylate to CBD and THC, respectively, during exposure to the elevated temperatures needed for extraction, vacuum distillation and even during prolonged storage at room temperature.

Accordingly, there is a need to further improve the existing technologies in terms of process simplicity, in order to guarantee compound integrity and obtain higher compound purity.

Furthermore, there is a related need to generate and isolate valuable intermediate compounds and provide novel cannabinoid derivatives that are accessible through the sought-after isolation and purification techniques.

The above objects are solved by the subject-matter of the claims and by the subject-matter described here below.

## Summary of the invention

In one aspect, the invention provides a compound according to Formula I

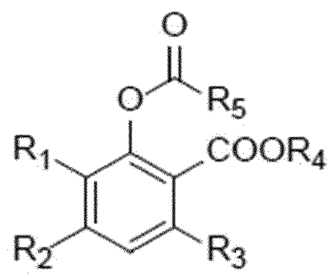

I

wherein $R^1$ = methyl-4-(prop-1-en-2-yl)-cyclohex-1-ene-3-yl (limonenyl);

wherein $R^2$ = acetate, propionate, butyrate, or OH;

wherein $R^3$ = propyl, pentyl, or heptyl;

wherein $R^4$ = methyl, ethyl, or propyl;

wherein $R^5$ = methyl, ethyl, or propyl,

or wherein $R^1$ = methyl-4-(2-propyl)-cyclohex-1-ene-3-yl or methyl-4-(2-propyl)-cyclohex-5-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ = methyl, ethyl, or propyl, and wherein $R^5$ = methyl, ethyl, or propyl,

or wherein $R^1$ = methyl-4-(2-propyl)-cyclohex-1-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ and $R^5$ vanish, i.e. are radicals forming a direct bond to each other.

The invention thus provides synthetic access to hitherto unknown cannabinoic acid derivatives which have been further converted into novel THC derivatives.

In a further aspect, the invention provides a process for the synthesis and/or purification of tetrahydrocannabinolic acid (THCA) alkanoate and/or cannabidiolic acid (CBDA) di-alkanoate, preferably tetrahydrocannabinolic acid (THCA) acetate and/or cannabidiolic acid (CBDA) di-acetate, comprising the steps of

- providing a crude plant extract containing a mixture of cannabinoic acids, or providing CBDA;
- contacting and/or reacting the crude plant extract or the CBDA with an acylation agent, preferably an acetylation agent, and a chemical base, to obtain THCA alkanoate and/or CBDA di-alkanoate, preferably THCA acetate and/or CBDA di-acetate; and
- isolating the obtained THCA alkanoate and/or CBDA di-alkanoate, preferably THCA acetate and/or CBDA di-acetate,

wherein the acylation agent is selected from the list of $C_1$-$C_{10}$ aliphatic or aromatic anhydrides, preferably acetic anhydride,

wherein the chemical base is preferably a tertiary amine, such as pyridine or preferably tri-ethylamine,

wherein isolation is carried out by means of crystallization.

This chemical method for the isolation of CBDA from crude plant extracts via the preparation of derivatives of CBDA and THCA is simple, efficient and also allows separating CBDA from a diverse mixture of chemical carboxylation products of CBD.

The method according to the invention circumvents time consuming and laborious chromatographic separations of cannabinoic acids by isolating the sought-after compounds through simple crystallization of chemically modified derivatives.

The method according to the invention is advantageous because it provides THCA acetate and CBDA di-acetate in pure and crystalline form which is and makes them distinct over many other cannabinoids which are oily and escape crystallization due to their chemical nature. Additionally, the method circumvents and/or solves the problem that THCA is labile and very prone to decarboxylation. Specifically, THCA acetate cannot decarboxylate and is much more stable than THCA.

In a further aspect, the invention provides a compound according to Formula I for use in medicine.

Cannabinoic acid derivatives have shown wide-ranging health benefits and applications in the medical field, and may serve to treat people and/or animals suffering from inflammation, nausea, convulsion, or even cancer.

Detailed description

**Novel derivatives of CBDA and THCA**

In one aspect, the invention provides a compound according to Formula I

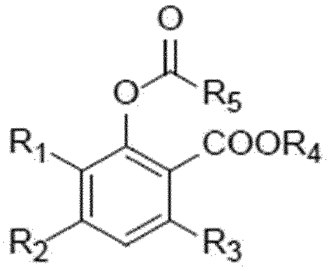

I

wherein $R^1$ = methyl-4-(prop-1-en-2-yl)-cyclohex-1-ene-3-yl (limonenyl);

wherein $R^2$ = acetate, propionate, butyrate, or OH;

wherein $R^3$ = propyl, pentyl, or heptyl;

wherein $R^4$ = methyl, ethyl, or propyl;

wherein $R^5$ = methyl, ethyl, or propyl,

or wherein $R^1$ = methyl-4-(2-propyl)-cyclohex-1-ene-3-yl or methyl-4-(2-propyl)-cyclohex-5-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ = methyl, ethyl, or propyl, and wherein $R^5$ = methyl, ethyl, or propyl,

or wherein $R^1$ = methyl-4-(2-propyl)-cyclohex-1-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ and $R^5$ vanish, i.e. are radicals forming a direct bond to each other.

The invention thus provides synthetic access to hitherto unknown cannabinoic acid derivatives which have been further converted into novel THC derivatives.

The provided compounds derive their utility from the fact that they are both crystalline and their decarboxylation is hindered through esterification of the phenolic alcohol adjacent to the carboxylic acid moiety and esterification of the carboxylic acid itself, which increases both their processability and stability. In contrast, the natural products with the acid functionality are labile and decompose at temperatures above 50 °C. The combined properties make them highly suitable with regard to cannabinoid extraction, purification and/or isolation.

In one embodiment, a compound is provided having a Formula I

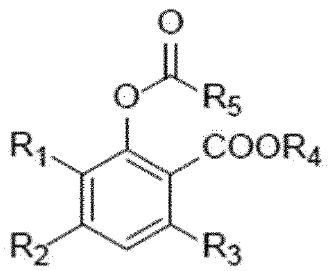

I

wherein $R^1$ = methyl-4-(prop-1-en-2-yl)-cyclohex-1-ene-3-yl (limonenyl);

wherein $R^2$ = acetate, or OH;

wherein $R^3$ = propyl, pentyl, or heptyl;

wherein $R^4$ = methyl;

wherein $R^5$ = methyl,

or wherein $R^1$ = methyl-4-(2-propyl)-cyclohex-1-ene-3-yl or methyl-4-(2-propyl)-cyclohex-5-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ = methyl, and wherein $R^5$ = methyl,

or wherein $R^1$ = methyl-4-(2-propyl)-cyclohex-1-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ and R5 vanish, i.e. are radicals forming a direct bond to each other.

The identified compounds are crystalline and thereby enable the purification and isolation of e.g. CBDA-di-acetate and further homologues according to Formula I from crude plant extracts. These compounds are value-added intermediates conducive to exceptionally high-yielding cyclisation and purity for the purpose of creating further derivatives, such as e.g. THCA, Tetrahydrocannabivarinic acid (THCVA), delta(9)-Tetrahydrocannabiphorolic acid (THCPA), THC, Tetrahydrocannabivarin (THCV), and Tetrahydrocannabiphorol (THCP).

In one embodiment, a compound is provided having Formula II

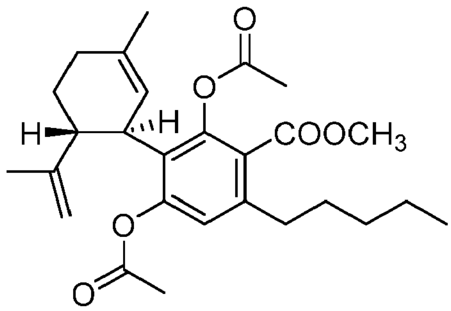

**II** (CBDA diacetate methyl ester).

In one embodiment, a compound is provided having Formula III

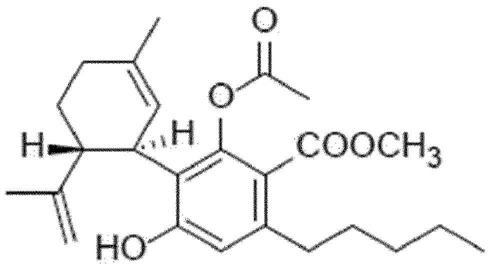

**III** (CBDA-2 acetate methyl ester).

The compound according to Formula III is valuable as it allows subsequent derivatization into a THCA derivative, i.e. compound according to Formula IVa.

In one embodiment, a compound is provided having Formula IVa

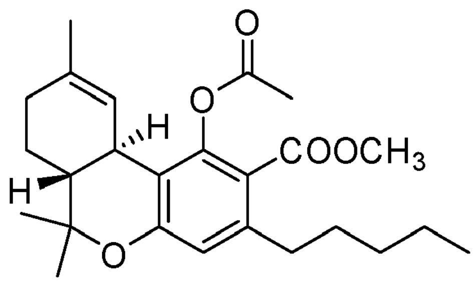

**IVa** (Delta-9-THCA acetate methyl ester).

In one embodiment, a compound is provided having Formula IVb

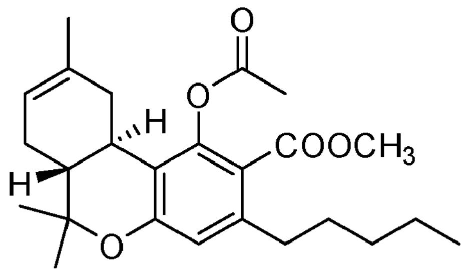

**IVb** (Delta-8-THCA acetate methyl ester).

In one embodiment, a compound is provided having Formula V

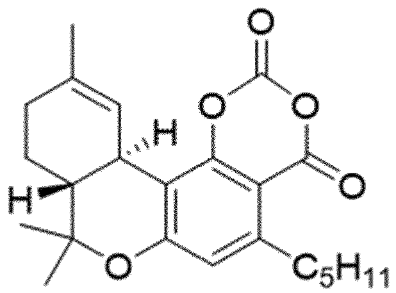

**V** (THCA carbonate).

The compound according to Formula **V** is highly valuable as it is a non-psychoactive derivative, which is further amenable to hydrolysis upon which it decomposes into THCA and $CO_2$. Hydrolysis of the compound according to Formula **V** can be effected by exposure to water or atmospheric moisture.

**Pharmaceutical composition**

In a further aspect, the invention provides a pharmaceutical composition comprising a compound according to Formula **I**

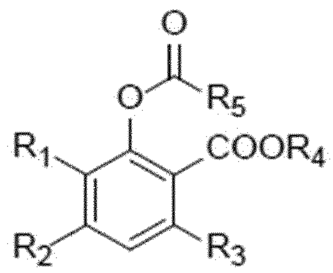

I

wherein $R^1$ = methyl-4-(prop-1-en-2-yl)-cyclohex-1-ene-3-yl (limonenyl);

wherein $R^2$ = acetate, propionate, butyrate, or OH;

wherein $R^3$ = propyl, pentyl, or heptyl;

wherein $R^4$ = methyl, ethyl, or propyl;

wherein $R^5$ = methyl, ethyl, or propyl,

or wherein $R^1$ = methyl-4-(2-propyl)-cyclohex-1-ene-3-yl or methyl-4-(2-propyl)-cyclohex-5-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ = methyl, ethyl, or propyl, and wherein $R^5$ = methyl, ethyl, or propyl,

or wherein $R^1$ = methyl-4-(2-propyl)-cyclohex-1-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ and $R^5$ vanish, i.e. are radicals forming a direct bond to each other,

in a pharmaceutically acceptable carrier.

In one embodiment, a pharmaceutical composition is provided comprising a compound selected from the list of

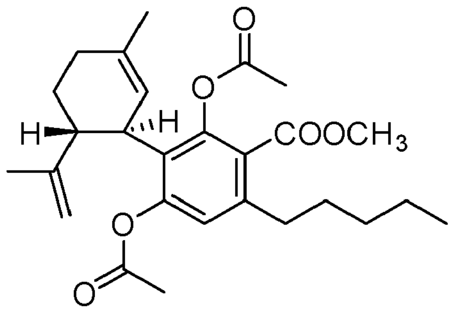

**II** (CBDA diacetate methyl ester),

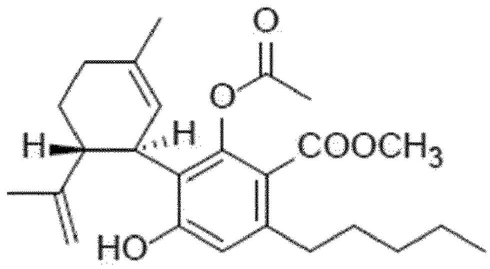

**III** (CBDA-2 acetate methyl ester),

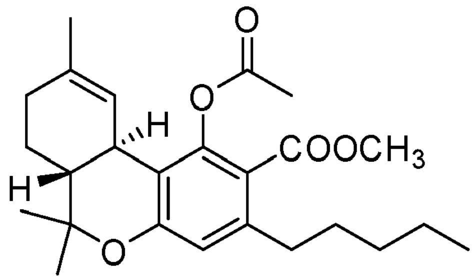

**IVa** (Delta-9-THCA acetate methyl ester),

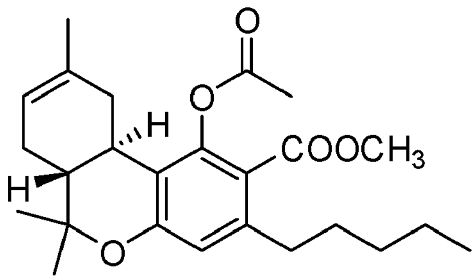

**IVb** (Delta-8-THCA acetate methyl ester), and

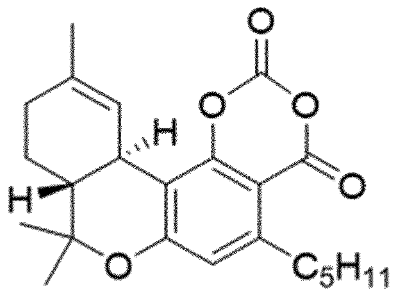

**V** (THCA carbonate),

in a pharmaceutically acceptable carrier.

**Process**

In a further aspect, the invention provides a process for the synthesis and/or purification of tetrahydrocannabinolic acid (THCA) alkanoate and/or cannabidiolic acid (CBDA) di-alkanoate, preferably tetrahydrocannabinolic acid (THCA) acetate and/or cannabidiolic acid (CBDA) di-acetate, comprising the steps of

- providing a crude plant extract containing a mixture of cannabinoic acids, or providing CBDA;
- contacting and/or reacting the crude plant extract or the CBDA with an acylation agent, preferably an acetylation agent, and a chemical base, to obtain THCA alkanoate and/or CBDA di-alkanoate, preferably THCA acetate and/or CBDA di-acetate; and
- isolating the obtained THCA alkanoate and/or CBDA di-alkanoate, preferably THCA acetate and/or CBDA di-acetate,

wherein the acylation agent is selected from the list of $C_1$-$C_{10}$ aliphatic or aromatic anhydrides, preferably acetic anhydride,

wherein the chemical base is preferably a tertiary amine, such as pyridine or preferably tri-ethylamine,

wherein isolation is carried out by means of crystallization.

This chemical method for the synthesis and/or purification of tetrahydrocannabinolic acid (THCA) alkanoate and/or cannabidiolic acid (CBDA) di-alkanoate allows the isolation of THCA and/or CBDA from crude plant extracts via the preparation of derivatives of CBDA and/or THCA. The process according to the invention is simple, efficient and also allows separating THCA and/or CBDA from a diverse mixture of chemical carboxylation products of for example CBD. Specifically, the process circumvents time consuming and laborious chromatographic separations of cannabinoic acids by isolating the sought-after compounds through crystallization of chemically modified derivatives.

The method according to the invention is advantageous because it provides THCA alkanoate and/or CBDA di-alkanoate, preferably THCA acetate and/or CBDA di-acetate, in pure and crystalline form which makes these compounds and their derivatives distinct over many other cannabinoids which are oily and escape crystallization due to their chemical nature. These acid-form alkanoates are protected from decarboxylation, and offer greater thermal stability for their manipulation and storage. In this respect, the method circumvents and/or solves the problem that THCA is labile and very prone to decarboxylation. Specifically, THCA acetate cannot decarboxylate and is much more stable than THCA.

If this disclosure refers to a step of "contacting and/or reacting" a crude plant extract or "reacting" or "treating" any other starting compound, including any intermediate compound, with a chemical agent or a number of chemical agents, optionally in a suitable solvent, it shall be understood that each step or sequence of steps is carried out for a time sufficient to effect (i.e. allow) the underlying reaction (to take place) of at least a portion of the starting compound(s) into the target (or intermediate) compound. Preferably, the time is sufficient to allow the underlying reaction to take place and to reach chemical equilibrium between the starting compound(s) and the target (or intermediate) compounds at the respectively chosen reaction or treatment conditions. Analogously, the skilled person understands and knows how to carry out the addition of chemical (re)agents under e.g. stirring and/or maintaining a preferred temperature in order to optimise and/or maximise the yields of the target compounds. Furthermore, the skilled person understands and knows how to carry out the work-up of reaction batches, e.g. adding water and/or combining with an aqueous HCl solution for neutralisation purposes, extraction using organic solvents, washing steps with e.g. aqueous HCl solution or with saturated NaCl solution, drying over $Na_2SO_4$, filtration and concentration, in order to optimise and/or maximise the yields of the target compounds.

In one embodiment, the crude plant extract is provided by enzymatic extraction. Advantageously, during an enzymatic extraction, not as much heat is generated so that decarboxylation-sensitive material is not decarboxylated.

In one embodiment, providing a crude plant extract containing a mixture of cannabinoic acids comprises providing hemp pollen, preferably by isolating the trichomes from the plant inflorescences optionally by a sieving process, and by subjecting the hemp pollen to an enzymatic process, wherein the cellulose of the plant cell walls is hydrolyzed and/or decomposed enzymatically to release cannabinoids into an aqueous solution, at a temperature between 10 and 40 °C, preferably between 20 and 30 °C. Advantageously, the enzymatic process preserves CBDA in its acid form and prevents it from decarboxylation.

In one embodiment, providing a crude plant extract containing a mixture of cannabinoic acids comprises providing hemp pollen, preferably by isolating the trichomes from the plant inflorescences optionally by a sieving process, by subjecting the hemp pollen to ultrasound accelerated maceration using alternating pressure and cavitation to effect at least partial cell decomposition and/or at least partial release of intracellular metabolic content, and by extracting the

cannabinoic acids using a solvent, preferably a chloroform/n-hexane (1:1, v/v) solvent mixture, at a temperature between 10 and 40 °C, preferably between 20 and 30 °C. Advantageously, the cold extraction process preserves CBDA in its acid form and prevents it from decarboxylation.

In one embodiment, the step extracting the cannabinoic acids using a solvent, preferably a chloroform/n-hexane (1:1, v/v) solvent mixture, is performed batch-wise and using per batch between 10 g to 50 g hemp pollen (HP) with an HP to solvent (or solvent mixture) ratio of between 1:4 and 1:6 (w/w) and employing three extraction cycles. Preferably each extraction cycle lasts at least 10 min, more preferably at least 30 min.

In one embodiment, contacting and/or reacting the crude plant extract with an acylation agent, preferably an acetylation agent, and a chemical base, wherein the acylation agent is selected from the list of $C_1$-$C_{10}$ aliphatic or aromatic anhydrides, preferably acetic anhydride, and wherein the chemical base is preferably a tertiary amine, such as pyridine or preferably tri-ethylamine, comprises dissolving the crude plant extract by shaking at room temperature in a suitable solvent, such as e.g. DMF, adding the acylation agent dropwise under stirring and maintaining the solvent temperature below 50°C, and allowing further contact and/or reaction for 1 to 4 hours, preferably 1.5 to 2.5 hours, at room temperature. This sequence of method steps provides for a synthesis to obtain THCA alkanoate and/or CBDA di-alkanoate, preferably THCA acetate and/or CBDA di-acetate.

If this disclosure refers to room temperature, a temperature range between 18 and 30 °C, preferably between 21 and 24 °C, shall be understood and employed.

In one embodiment, the acylation agent is selected from the list of $C_1$-$C_{10}$ aliphatic or aromatic anhydrides, such as e.g. propionic anhydride, butyric anhydride, pentanoic anhydride, hexanoic anhydride, heptanoic anhydride, octanoic anhydride, nonaoic anhydride, decanoic anhydride, benzoyl anhydride and substituted benzoyl anhydride, preferably acetic anhydride.

In one embodiment, the chemical base is a tertiary amine, such as e.g. pyridine, diisopropylethylamine or preferably triethylamine. Advantageously, triethylamine is inexpensive, easy to remove from the reaction mixture, and has further benefits over many chemical bases that may be used for the process, such as e.g. pyridine.

In one embodiment of the process, a further step may be included after the step contacting and/or reacting the crude plant extract with an acylation agent and a chemical base, comprising cooling to 0°C, adding water to the contacted and/or reacted crude plant extract, e.g. the reaction mixture, under stirring for 0.5 h at room temperature, combining with an aqueous HCl solution (around 2 M), extracting twice with ethyl acetate, washing the combined ethyl acetate extracts with aqueous HCl solution (around 2 M), followed by washing three times with saturated NaCl solution, drying over $Na_2SO_4$, filtration and concentration.

In one embodiment of the process, isolating the obtained THCA alkanoate and/or CBDA di-alkanoate, preferably THCA acetate and/or CBDA di-acetate, is carried out by means of crystallization. The skilled artisan knows and understands that crystallization encompasses complete dissolution of a crude product or material in a solvent, preferably by heating the solvent to effect and/or accelerate dissolution but establishing and/or maintaining a temperature below the decomposition temperature of the crude product or material, optional filtration through e.g. a layer of silica gel, and subsequent cooling to obtain crystals of the product or material.

Advantageously, THCA alkanoates and/or CBDA di-alkanoates, preferably THCA acetate and/or CBDA di-acetate, are amenable to crystallisation, whereas most other cannabinoid compounds and derivatives are oily, difficult to work with and difficult to process. In particular, the crystallisation of THCA alkanoates and/or CBDA di-alkanoates, preferably THCA acetate and/or CBDA di-acetate, is feasible and simple.

With respect to CBDA and THCA, the free non-derivatised acids cannot be crystallised, because heating would lead to decarboxylation. Advantageously, THCA alkanoates and/or CBDA di-alkanoates, preferably THCA acetate and/or CBDA di-acetate, can be crystallised without decarboxylation, and to thus leave intact the chemical derivative.

In one embodiment of the process, crystallization is performed in n-pentane or n-hexane. Advantageously, the low boiling points of n-pentane or n-hexane, in particular n-pentane, provide for and support crystallisation conditions that leave the derived compounds, i.e. THCA alkanoates and/or CBDA di-alkanoates, preferably THCA acetate and/or CBDA di-acetate, intact, and may thereby inherently prevent decay reactions, such as decarboxylation.

In one embodiment, a process for the synthesis and/or purification of tetrahydrocannabinolic acid (THCA) alkanoate, preferably tetrahydrocannabinolic acid (THCA) acetate, is provided comprising the steps of

- providing a crude plant extract containing a mixture of cannabinoic acids;
- contacting and/or reacting the crude plant extract with an acylation agent, preferably an acetylation agent, and a chemical base, to obtain THCA alkanoate, preferably THCA acetate; and
- isolating the obtained THCA alkanoate, preferably THCA acetate,

wherein the acylation agent is selected from the list of $C_1$-$C_{10}$ aliphatic or aromatic anhydrides, preferably acetic

anhydride,

wherein the chemical base is preferably a tertiary amine,

wherein isolation is carried out by means of crystallization.

In one embodiment, a process for the synthesis and/or purification of cannabidiolic acid (CBDA) di-alkanoate, preferably cannabidiolic acid (CBDA) di-acetate, is provided comprising the steps of

- providing CBDA;
- contacting and/or reacting the CBDA with an acylation agent, preferably an acetylation agent, and a chemical base, to obtain CBDA di-alkanoate, preferably CBDA di-acetate; and
- isolating the obtained CBDA di-alkanoate, preferably CBDA di-acetate,

wherein the acylation agent is selected from the list of $C_1$-$C_{10}$ aliphatic or aromatic anhydrides, preferably acetic anhydride,

wherein the chemical base is preferably a tertiary amine,

wherein isolation is carried out by means of crystallization.

In one embodiment, the process comprises the further step of treating the obtained CBDA di-acetate with an alkylation agent, such as e.g. $C_1$-$C_{10}$ dialkylsulfate, preferably dimethyl sulfate, to obtain a first CBDA di-acetate derivative, preferably CBDA diacetate methyl ester according to Formula II

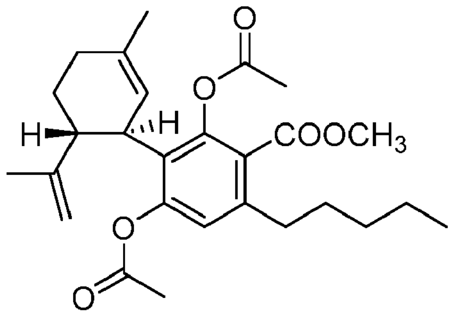

**II.**

In one embodiment, the step of treating the obtained CBDA di-acetate with an alkylation agent comprises dissolving CBDA-di-acetate in a solvent, preferably acetonitrile, and adding an excess of triethylamine, preferably 1.1 mol equivalents with respect to CBDA-di-acetate, while stirring the solution at room temperature, drop-wise adding an excess of dimethyl sulfate, preferably 1.1 mol equivalents with respect to CBDA-di-acetate, into the stirred solution, maintaining a temperature at or below 50°C, further stirring the solution at room temperature.

In an alternative embodiment, the step of treating the obtained CBDA di-acetate with an alkylation agent comprises dissolving CBDA-di-acetate in a solvent, preferably acetonitrile, and adding an excess of triethylamine, preferably 1.1 mol equivalents with respect to CBDA-di-acetate, while stirring the solution at room temperature, drop-wise adding an excess of dimethyl sulfate, preferably 1.1 mol equivalents with respect to CBDA-di-acetate, into the stirred solution, maintaining a temperature at around 60°C for 2 h, and pouring the reaction solution into ice-cold 1 M aqueous HCl solution, and extracting the resulting suspension twice with dichloromethane, washing the combined organic extracts with 1 M aqueous HCl solution, saturated aqueous Na-$HCO_3$ solution and saturated aqueous NaCL solution and drying over $Na_2SO_4$.

In one embodiment, the process comprises the further step of deacylating the first CBDA di-acetate derivative to obtain a CBDA mono-acetate derivative, preferably CBDA-2-acetate methyl ester according to Formula III

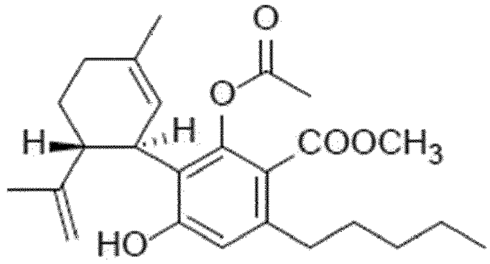

III.

In one embodiment, the step of deacylating the first CBDA di-acetate derivative comprises dissolving the first CBDA di-acetate derivative in an alcohol, e.g. methanol, adding a base reagent, e.g. sodium methanolate or a solution of ammonia in methanol, and stirring the solution at room temperature for 24 h. In one embodiment relying on sodium methanolate, the process comprises the further steps of neutralizing the solution by addition of Amberlyst IR 120 ion exchange resin (in its protonated form), filtration and concentration. In one embodiment relying on ammonia, the process comprises the further steps of concentrating the solution.

In one embodiment, the process comprises the further step of converting the CBDA mono-acetate derivative into a THCA mono-acetate derivative, preferably THCA 2-acetate methyl ester according to Formula **IVa**

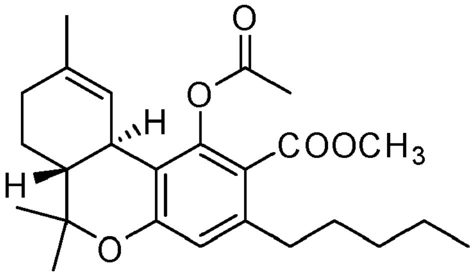

**IVa**.

In one embodiment, the step of converting the CBDA mono-acetate derivative into a THCA mono-acetate derivative comprises dissolving the CBDA mono-acetate derivative in dichloromethane and adding boron trifluoride diethyl etherate, stirring under a nitrogen atmosphere, and washing with saturated aqueous $NaHCO_3$ solution, 2 N aqueous HCl solution, saturated aqueous NaCl solution, drying over $Na_2SO_4$ and filtration. In a further embodiment, the process comprises the additional steps of concentrating the filtrate and recrystallization from n-hexane.

In one embodiment, the process comprises the further step of converting THCA 2-acetate methyl ester, preferably via a THCA intermediate, into THCA carbonate according to Formula **V**

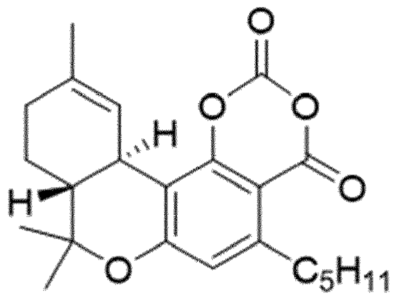

**V**.

In one embodiment, the step of converting THCA 2-acetate methyl ester comprises dissolving and stirring

THCA 2-acetate methyl ester in a solution of 0.5 to 2.0 M LiOH, preferably about 1 M LiOH, in an alcohol, preferably methanol, for 12 to 36 h, preferably about 24 h. In a further embodiment, the process comprises the additional steps of reacting with an aqueous HCl solution, preferably of 2 M concentration, extracting twice with dichloromethane, and washing the combined extracts with saturated aqueous NaCl solution, drying over $Na_2SO_4$, filtration and concentration to obtain crude THCA.

In a further embodiment, the process comprises the steps of dissolving the crude THCA in an aromatic solvent, such as e.g. toluene, adding a base reagent, e.g. trimethylamine and phosgene, preferably 2 M phosgene, stirred the solution at room temperature, washing with saturated aqueous $NaHCO_3$ solution, drying over $Na_2SO_4$, filtration and concentration, to obtain THCA carbonate.

In one embodiment of the process, the obtained derivatives are at least 70% pure, preferably 90% pure, and more preferably 99% pure, wherein the obtained derivatives are selected from

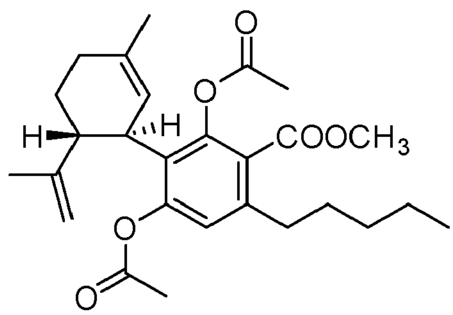

**II** (CBDA diacetate methyl ester),

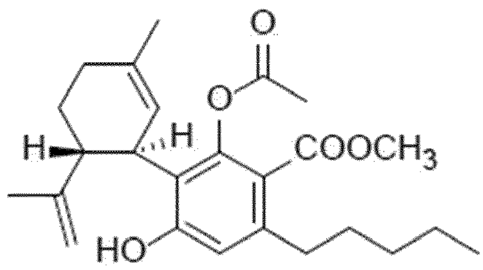

**III** (CBDA-2 acetate methyl ester),

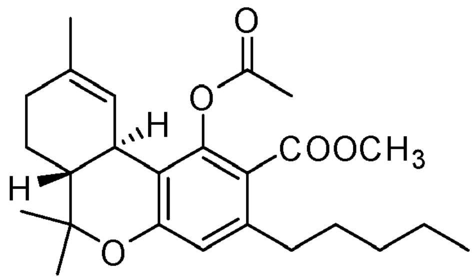

**IVa** (Delta-9-THCA acetate methyl ester),

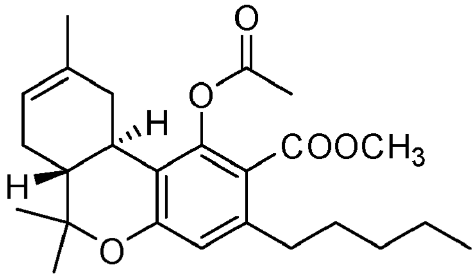

**IVb** (Delta-8-THCA acetate methyl ester), and

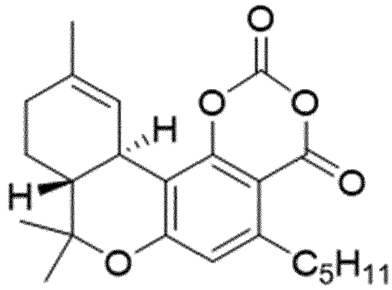

**V** (THCA carbonate).

In one embodiment of the process, a distillation step is absent and/or a chromatography step is absent. Advantageously, chromatography steps are avoided because such measures are tedious and expensive. Advantageously, also a distillation step is absent because the compounds described and provided herein are heat-labile and may suffer from decomposition at increased temperatures, with reference to room temperature, preferably with reference to room temperature plus 20 °C. In particular, the absence of a distillation step may prevent decarboxylation of THCA alkanoate and/or CBDA di-alkanoate, preferably THCA acetate and/or CBDA di-acetate, during or as part of the process.

In one embodiment of the process, the plant extract is obtained from a plant of the species *Cannabinaceae,* preferably a plant selected from the list of *Cannabis sativa*, *Cannabis indica*, and *Cannabis ruderalis.* Plant extracts obtained from the named plant species are naturally rich in cannabinoids and thus lend themselves to provide for the synthesis and/or purification of THCA alkanoate and/or CBDA di-alkanoate, preferably THCA acetate and/or CBDA di-acetate.

**Medical use**

In a further aspect, the invention provides a compound according to Formula **I** for use in medicine,

wherein $R^1$ = methyl-4-(prop-1-en-2-yl)-cyclohex-1-ene-3-yl (limonenyl);

wherein $R^2$ = acetate, propionate, butyrate, or OH;

wherein $R^3$ = propyl, pentyl, or heptyl;

wherein $R^4$ = methyl, ethyl, or propyl;

wherein $R^5$ = methyl, ethyl, or propyl,

or wherein $R^1$ = methyl-4-(2-propyl)-cyclohex-1-ene-3-yl or methyl-4-(2-propyl)-cyclohex-5-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ = methyl, ethyl, or propyl, and wherein $R^5$ = methyl, ethyl, or propyl,

or wherein $R^1$ = methyl-4-(2-propyl)-cyclohex-1-ene-3-yl, wherein $R^2$ = O, and $R^1$ and $R^2$ together form a ring

structure in which $R^2$ is an internal ring atom, wherein $R^3$ = propyl, pentyl, or heptyl, wherein $R^4$ and $R^5$ vanish, i.e. are radicals forming a direct bond to each other.

In a further aspect, the invention provides a compound according to Formula **II** for use in medicine.
In a further aspect, the invention provides a compound according to Formula **III** for use in medicine.
In a further aspect, the invention provides a compound according to Formula **IVa** for use in medicine.
In a further aspect, the invention provides a compound according to Formula **IVb** for use in medicine.
In a further aspect, the invention provides a compound according to Formula **V** for use in medicine.
Cannabinoic acid derivatives have shown wide-ranging health benefits and applications in the medical field, and may serve to treat people and/or animals suffering from inflammation, nausea, convulsion, or even cancer.
In one embodiment, a compound according to Formula **I** is provided for use in treating or pre-venting a disease associated with a cannabinoid receptor in a subject in need thereof, wherein the cannabinoid receptor is one or more of CB1, CB2, 5HT1A, 5HT2A, GPR18, GPR55, GPRI 19, TRPVI, TPRV2, PPARy or a $\mu$-opioid receptor.
In one embodiment, a compound according to Formula **II**, Formula **III**, Formula **IVa**, Formula **IVb**, or Formula **V** is provided for use in treating or preventing a disease associated with a cannabinoid receptor in a subject in need thereof, wherein the cannabinoid receptor is one or more of CB1, CB2, 5HT1A, 5HT2A, GPR18, GPR55, GPRI 19, TRPVI, TPRV2, PPARy or a $\mu$-opioid receptor.
In one embodiment, a compound according to Formula I is provided for use in treating or pre-venting a disease associated with a cannabinoid receptor in a subject in need thereof, wherein the cannabinoid receptor is CB1 and/or CB2.
In one embodiment, a compound according to Formula **II,** Formula **III,** Formula **IVa**, Formula **IVb**, or Formula **V** is provided for use in treating or pre-venting a disease associated with a cannabinoid receptor in a subject in need thereof, wherein the cannabinoid receptor is CB1 and/or CB2.
Cannabinoids, as well as their derivatives, are compounds active on cannabinoid receptors in humans and animals and have been implicated in many of the pharmacological benefits underlying the medical conditions, such as chronic pain, epilepsy, sleep disorders, anxiety, cancer, glaucoma, nausea, amyotrophic lateral sclerosis (ALS), Alzheimer’s disease, Crohn’s disease, Post-traumatic Stress Disorder (PTSD), arthritis, and fibromyalgia.
CB1 and CB2 receptors, as well as other receptors have been implicated in modulating the activity of cannabinoids in the human and/or animal body. Serotonin receptors, such as 5HT1A and 5HT2A, are known targets of cannabinoids.
Both receptor classes, i.e. CB1/CB2 receptors and the serotonin receptors, have been implicated with several neurological phenomena, such as anxiety, appetite, cognition, and mood. Further receptors, such as e.g. GPR18, GPR55, GPR119, TRPV1, TPRV2, PPARs, and $\mu$-opioid receptors are regulated and/or influenced to at least some extent by cannabinoid or cannabinoid-derived compounds.

Examples

**Extraction of crude plant material**

The crude plant extract is prepared via an enzymatic polysaccharide hydrolyzing process (Herbolea) or a "cold" extraction process, both processes preserving CBDA from decarboxylation.

Method 1

Dry hemp (25 g) was ground in a mortar and suspended in n-hexane (1 l). The mixture was stirred at room temperature for 3 h followed by sonification in an ultrasound bath for 15 min. The resulting slurry was filtered through a paper filter and the filtrate was concentrated in vacuo at a rotary evaporator (bath temperature 30 °C) to ca. 200 ml. The light red solution was extracted with 1% aqueous NaOH solution (150 ml) and the alkaline aqueous extract was poured into ice cold 10% HCl solution (200 ml). The resulting mixture was extracted with ethyl acetate (2 x 50 ml), the organic extracts washed with saturated NaCl solution, dried over $Na_2SO_4$, filtered and concentrated.

Method 2

Hemp pollen (HP) is a resin produced by isolating the trichomes from the plant inflorescences by a sieving process and was used as a CBDA source. Hemp pollen, obtained from Hemp Farm Lab farmers (Caserta, Italy), was subjected to ultrasound accelerated maceration (Branson Ul-trasonics™ M3800-E, Danbury, CT, USA). Alternating pressure and cavitation was used for cell decomposition and the release of the intracellular metabolic content. A chloroform/n-hexane (1:1, v/v) solvent mixture was used as extracting solution, using per batch around 10 g HP with an HP to solvent mixture ratio of 1:5 and three extraction cycles. Each extraction cycle lasted 30 min. The obtained compound

mixture was enriched with cannabinoids at a yield of at least 25%.

Method 3

150 g of dried industrial hemp buds were placed in a kitchen aid mixer set at 100 rpm along with 340 g water, 100 g olive oil, 6 g citric acid monohydrate and a mixture of commercial food-grade enzymes consisting of: Celluclast 1.5 L (cellulase), Ultraflow Max (betaglucanase), Peclyve (pectinase, beta-glucanases, cellulases, and beta-mannanases) and Ceremix 2XL (Alpha-amylase, Beta-glucanase, Protease). Total enzymes concentration was 3% of the hemp plant material weight. After 3.5 hours, the mixture becomes homogenous. Subsequent centrifugation of the mixture (11,000 rpm for 5 min) provided 91 g of lipid-soluble extract, 80 g of an intermediate aqueous phase and 410 g of a wet solid fraction.

The lipid-soluble extract was then washed with 0.1 N aqueous NaOH whereupon the lipid phase is discarded and the aqueous phase is then neutralized with 2 N aqueous HCl after which the crude CBDA precipitate is filtered out and centrifuged to provide a crude oleaginous CBDA of approx. 70% purity.

**Synthesis and compound characterization**

All reactions were performed under an atmosphere of nitrogen using solvents dried by standard procedures. Reaction progress was monitored by TLC on Polygram SIL G/UV254 silica gel plates from Macherey & Nagel and on Silicagel plates 60 RP-18 F254 from Supelco. Detection of spots was effected by charring with sulfuric acid (5% in ethanol), staining by spraying the plates with an alkaline aqueous solution of potassium permanganate, staining plates in a iodine chamber or by inspection of the TLC plates under UV light (254 nm).

Preparative chromatography was performed on silica gel (0.032-0.063 mm) from Macherey & Nagel.

NMR spectra were recorded with the following spectrometers: Bruker Avance III HD 400 (1H: 400.2 MHz; 13C: 100.6 MHz), Bruker Avance III HDX 600 (1H: 600.2 MHz; 13C: 150.9 MHz) and Bruker Avance III HDX 700 (1H: 700.3 MHz; 13C: 176.1 MHz); and calibrated for the solvent signal ($^1$H: $CDCl_3$: $\delta$ = 7.26 ppm; acetone-$d_6$: $\delta$ = 2.05 ppm; dichlormethane-$d_2$: $\delta$ = 5.32 ppm; DMSO-$d_6$: $\delta$ = 2.50 ppm; $^{13}$C: $CDCl_3$: $\delta$ =77.16 ppm; acetone-$d_6$: $\delta$ = 29.92 ppm; dichlor-methane-$d_2$: $\delta$ = 54.0 ppm; DMSO-$d_6$: $\delta$ = 39.51 ppm).

ESI-TOF-HRM spectrometry was performed on a Bruker MAXIS 4G spectrometer.

Elemental analyses were obtained from a HEKAtech Euro EA 3000 apparatus.

Optical rotations were determined with a Perkin-Elmer Polarimeter 341 in a 10 cm cuvette at 20 °C with a wavelength of 589 nm (Na-lamp).

Melting points were measured with a Büchi Melting Point M-560 apparatus.

Example 1: Isolation of CBDA-di-acetate from hemp extract

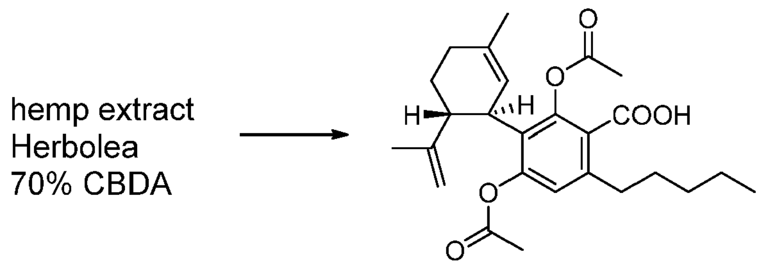


Five grams of Herbolea extract containing ca. 70% CBDA (w/w) was dissolved with shaking at room temperature in a mixture of DMF (10 ml) and triethylamine (10 ml) to give a deep red solution. Acetic anhydride (10 ml) was added dropwise to the stirred solution so that the temperature does not exceed 50°C. The mixture was stirred for 2 h at room temperature and cooled to 0°C. Water (10 ml) was added and the mixture was stirred for 0.5 h at room temperature. The mixture was poured into aqueous HCl solution (2 M, 50 ml) and extracted with ethyl acetate (2 $\times$ 35 ml). The combined ethyl acetate extracts were washed with aqueous HCl solution (2 M, 1 $\times$ 50 ml) followed by saturated NaCl solution (3 x 50 ml), dried over $Na_2SO_4$, filtered and concentrated. The brown oily residue (5.47 g) was dissolved in n-hexane/diethyl ether or alternatively in n-hexane/t-butyl methyl ether (1/1 v/v, 50 ml) and filtered through a layer of silica gel. The filtrate was concentrated and the oily residue was taken up with boiling (n-hexane (30 ml). After removal of heat, crystallization immediately occurs to afford CBDA-di-acetate (4.01 g, 93%) as white needles.

The melting point of CBDA-di-acetate was determined as 127°C (literature value: 126-128°C, R. Mechoulam, Z. Ben-Zvi, Chem. Commun. 1969, 343-344).

Compound identity was confirmed by high-resolution mass spectrometry (HRMS): theoretical $C_{26}H_{33}O_5$ $[M+Na]^+$ m/z = 465.22476; found: m/z = 465.22486. Compound identity was confirmed by elemental analysis: calculated for $C_{26}H_{33}O_5$: C 70.56, H 7.74; found: C 70.55, H 7.62.

The optical rotation was measured as $[a]_D^{20}$ = -68° (c = 10 mg / 1.5 ml; in $CHCl_3$).

$^1$H-NMR spectra and $^{13}$C-NMR spectra were recorded and spectral data were extracted:

$^1$H-NMR ($CDCl_3$): δ=0.80 (t, 3 H, J=6.6 Hz, H-5"), 1.24, 1.25 (2 d, 4 H, H-3", 4"), 1.52 (s, 3 H, $COCH_3$), 1.60 (s, 3 H, $COCH_3$), 1.52 - 1.71 (m, 8 H, H-2", 7', 10'), 1.96 (br.d (broad doublet), 1 H, H-4a'), 2.14 (br.s (broad singlet), 3 H, H-4', 5b'), 2.61 (t, 2 H, H-1"), 2.73 (br.s, 1 H, H-2'), 3.39 (br.s, 1 H, H-1'), 4.39, 4.49 (2 s, 2 H, H-9'), 5.13 (s, 1 H, H-6'), 6.77 (s, 1 H, H-5), 7.19 (s, 1 H, COOH).

$^{13}$C-NMR ($CDCl_3$): δ=14.0 (C-5"), 20.7, 20.9 (4C, C-7', 10', $C_{Ac}$), 22.4 (C-4"), 28.6 (C-3"), 30.4 (C-3'), 30.7, 31.7 (C-1",2"), 33.7 (C-4'), 38.7 (C-1'), 45.5 (C-2'), 111.5 (C-9'), 123.9 (C-5), 128.0 (C-5'), 133.4 (C-1), 142.0 (C-3), 147.3 (C-4), 148.5 (C-8'), 151.4 (C-2).

**Example 2: Isolation of CBDA-di-acetate via carboxylation of CBD**

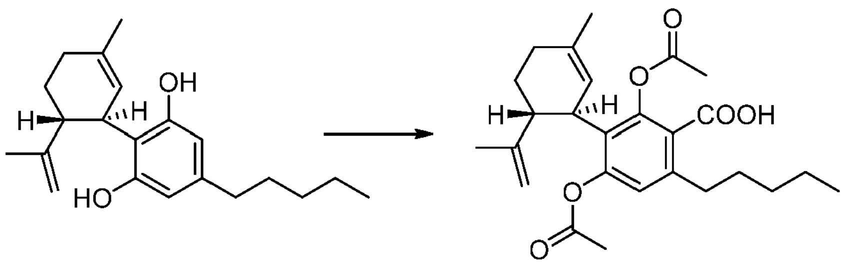

CBD (3.14 g, 10.0 mmol, purchased from American-Alb Hemp Company) was dissolved in DMF (10 ml) and magnesium methyl carbonate solution in DMF (2 M, 12.5 ml, 25.0 mmol) was added. The mixture was stirred at 130°C for 4 h according to the procedure of R. Mechoulam, Z. Ben-Zvi, Chem. Commun. 1969, 343-344. Water (5 ml) was added, the resulting mixture was stirred for 1 h at room temperature, poured into water (100 ml) and extracted with diethyl ether (3 x 50 ml). The combined diethyl ether extracts were dried with Na2SO4, filtered and concentrated to afford a mixture of CBD and CBDA containing ca. 75% CBDA. The crude CBD/CBDA mixture was treated with acetic anhydride as described in Example 1 to afford CBDA-di-acetate (3.05 g, 69%).

**Example 3: Methylation of CBDA-di-acetate (novel compound)**

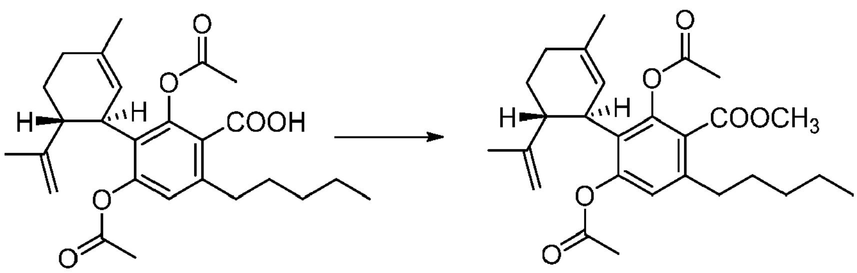

CBDA-di-acetate (10 g, 22.6 mmol) was dissolved in acetonitrile (50 ml) and triethylamine (2,52 g or 3,46 ml, 24,9 mmol, 1,1 mol-equivalents) was added with stirring at room temperature. Dimethyl sulfate (3,14 g or 2,4 ml, 24,9 mmol, 1,1 mol-equivalents) was dropped into the stirred mixture at such a rate that the temperature does not exceed 50°C, and the mixture was stirred at room temperature until TLC (RP-18 silica gel, MeOH/acetic acid 19/1) showed

complete conversion of the starting material (16 h). Alternatively, the mixture can be stirred at 60°C for 2 h.

The mixture was poured into ice cold 1 M aqueous HCl solution (250 ml) and the resulting suspension was extracted with dichloromethane (2 × 50 ml). The combined organic extracts were successively washed with 1 M aqueous HCl solution (1 × 100 ml), saturated aqueous $NaHCO_3$ solution (2 x 100 ml) and brine (1 × 100 ml) and dried over $Na_2SO_4$. Filtration and concentration afforded CBDA-di-acetate-methyl-ester as a slightly yellow oil (9,8 g, 95%).

Compound identity was confirmed by high-resolution mass spectrometry (HRMS): theoretical $C_{27}H_{36}O_6$ $[M+Na]^+$ m/z = 479.24041; found: m/z = 479.24105.

The optical rotation was measured as $[a]_D^{20}$ = -67° (c = 1.6 g / 100 ml, $CHCl_3$).

$^1$H-NMR spectra and $^{13}$C-NMR spectra were recorded and spectral data were extracted:

$^1$H-NMR ($CDCl_3$): δ=0.91 (t, 3 H, J=6.5 Hz, H-5"), 1.33 (br. s., 4 H, H-3", 4"), 1.61 (s, 3 H, $COCH_3$), 1.70 (s, 3 H, $COCH_3$), 1.56-1.84 (m, 2 H, H-2"), 2.02-2.23 (m, 11 H, H-1', 3', 4', 7', 10'), 2.61 (br. s, 1 H, H-2'), 2.70 (t, 2 H, J=3.9 Hz, H-1"), 3.47 (br. s, 1 H, H-1'), 3.87 (s, 3 H, $OCH_3$), 4.48, 4.58 (2 s, 2 H, H-9'), 5.23 (s, 1 H, H-6'), 6.84 (s, 1 H, H-5).

$^{13}$C-NMR ($CDCl_3$): δ=14.0 (C-5"), 20.7, 20.9 (2C, $CH_{3Ac}$), 22.4 (C-4"), 23.4 (C-7'), 28.6 (C-3"), 30.5 (2 C, C-2", 3'), 31.7 (C-1"), 33.6 (C-4'), 38.7 (C-10'), 45.5 (C-2'), 52.2 ($OCH_3$), 111.5 (C-9'), 124.0 (C-5'), 133.2 (C-1), 141.0 (C-3), 147.3 (C-5), 148.0 (C-4), 150.8 (2 C, C-2, 8'), 167.1, 168.7 (C=O).

**Example 4: Deacetylation of CBDA-di-acetate methyl ester (novel compound)**

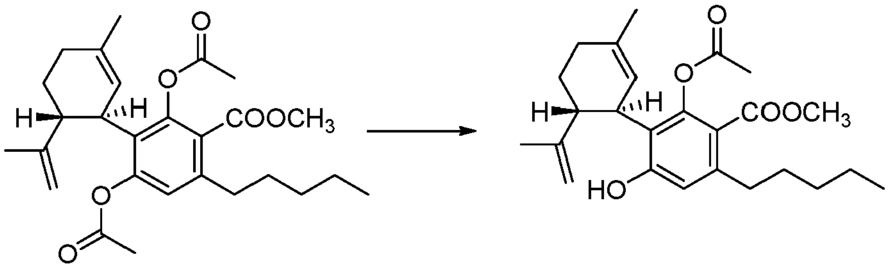

CBDA-di-acetate-methyl-ester (4.57 g, 10.0 mmol) was dissolved in methanol (20 ml), sodium methanolate (54 mg, 1.0 mmol) or a 1 M solution of ammonia in methanol (2.0 ml, 2.0 mmol ammonia) was added and the solution was stirred at room temperature for 24 h. In case of sodium methanolate, the solution was neutralized by the addition of Amberlyst IR 120 ion exchange resin (H+ form), filtered and concentrated. In case of ammonia, the solution was directly concentrated to afford the mono-deacetylated CBDA-2-acetate methyl ester (4.15 g, 100%).

Compound identity was confirmed by high-resolution mass spectrometry (HRMS): theoretical $C_{25}H_{34}O_5$ $[M+Na]^+$ m/z = 437.23039; found: m/z = 437.23027.

The optical rotation was measured as $[a]_D^{20}$ = -16.2° (c = 0.7 g / 100 ml, $CHCl_3$)

$^1$H-NMR spectra and $^{13}$C-NMR spectra were recorded and spectral data were extracted:

$^1$H-NMR ($CDCl_3$): δ=0.80 (t, 3 H, J=6.5 Hz, H-5"), 1.23 (br. s, 4 H, H-3", 4"), 1.53 (s, 3 H, $COCH_3$), 1.44-1.70 (m, 2 H, H-2"), 1.99-2.21 (m, 11 H, H-1', 3'; 4', 7', 10'), 2.40-2.66 (m, 2 H, H-1", 2'), 2.70, 3.47 (br. s, 1 H, H-1'), 3.75 (s, 3 H, $OCH_3$), 4.33, 4.53 (2 s, 2 H, H-9'), 5.47 (s, 1 H, H-6'), 6.17 (s, 1 H, H-5), 6.51 (s, 1 H, OH).

$^{13}$C-NMR ($CDCl_3$): δ=14.0 (C-5"), 20.7 ($CH_{3Ac}$), 22.5 (C-4"), 23.7 (C-7'), 27.7 (C-3"), 30.8 (2 C, C-2", 3'), 31.8 (C-1"), 33.8 (C-4'), 38.1 (C-10'), 45.6 (C-2'), 52.0 ($OCH_3$), 112.0 (C-9'), 122.6 (C-5'), 135.2 (C-1), 141.7 (C-3), 142.6 (C-5), 146.5 (C-4), 155.9, 157.1 (C-2, 8'), 167.1, 167.4 (C=O).

**Example 5a: Cyclization of CBDA-acetate methyl ester to Delta-9-THCA-acetate methyl ester (novel compound)**

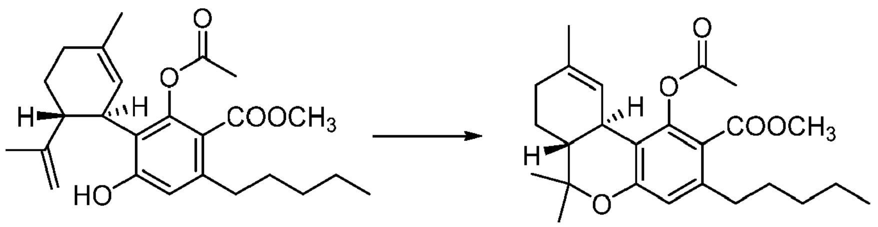

A solution of CBDA-2-acetate methyl ester (2.07 g, 5.0 mmol), tetraethylene glycol dimethylether (0.24 ml), diisopropylethylamine (0.24 ml) and boron trifluoride diethyl etherate (0.40 ml) in dichloromethane (50 ml) was stirred under an atmosphere of nitrogen until TLC shows complete consumption of the starting material (24 h). The solution was successively washed with saturated aqueous $NaHCO_3$ solution, 2 N aqueous HCl solution, saturated aqueous NaCl solution, dried over $Na_2SO_4$ and filtered. Concentration of the filtrate and recrystallization of the residue from n-hexane gave Delta-9-THCA-acetate methyl ester (2.0 g, 97%).

Compound identity was confirmed by high-resolution mass spectrometry (HRMS): theoretical $C_{25}H_{34}O_5$ $[M+Na]^+$ m/z = 437.22984; found: m/z = 437.22970

The optical rotation was measured as $[a]_D^{20} = -185°$ (c = 0.9 g / 100 ml, $CHCl_3$).

$^1$H-NMR spectra and $^{13}$C-NMR spectra were recorded and spectral data were extracted:

$^1$H-NMR ($CDCl_3$): δ=0.80 (t, 3 H, J=7.0 Hz, H-5"), 1.19-1.42 (m, 4 H, H-3",4"), 1.43-1.53 (m, 2 H, H-2"), 1.53 (s, 3 H, H-9'), 1.62-1.74 (m, 4 H, H-6',10'), 1.70 (s, 3 H, H-7'), 2.02 (br. s, 2 H, H-4'), 2.14 (s, 3 H, $CH_3C$=O), 2.41-2.49 (m, 4 H, H-1",5'), 2.62 (br. s, 1 H, H-1'), 3.74 (s, 3 H, $OCH_3$), 6.21 (s, 1 H, H-5), 6.53 (s, 1 H, H-2').

$^{13}$C-NMR ($CDCl_3$): δ=14.0 (C-5"), 19.4 (C-9'), 20.7 ($CH_{3Ac}$), 22.5 (C-4"), 23.7 (C-10'), 27.7 (C-5'), 30.1 (C-4'), 30.8 (C-3"), 31.8 (C-2"), 33.8 (C-1"), 38.1 (C-1'), 45.6 (C-6'), 52.0 ($OCH_3$), 77.4 (C-8'), 116.0 (C-5), 117.9 (C-1), 120.5 (C-3), 122.7 (C-2'), 141.0 (C-3'), 142.6 (C-2), 146.4 (C-4), 157.1 (C-6), 167.4 (COO), 169.1 (C=OAc).

**Example 5b: Cyclization of CBDA-acetate methyl ester to Delta-8-THCA-acetate methyl ester (novel compound)**

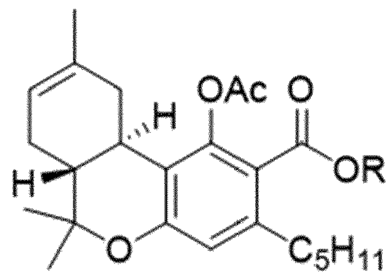

R=Me unknown

CBDA-acetate methyl ester (2.07 g, 5.0 mmol) was dissolved in toluene (10 ml), toluene-4-sulfonic acid monohydrate (10 mg, 0.05 mmol) was added and the solution heated to 80 °C with stirring for 3 h. The solution was then allowed to cool to room temperature and washed with saturated aqueous sodium hydrogen carbonate. The organic phase was then separated, dried with anhydrous sodium sulfate, filtered, and evaporated to dryness providing Delta-8 THCA-methyl ester (1.98 g, 96%).

**Example 6: Conversion of the THCA-acetate methyl ester into THCA-carbonate (novel compound)**

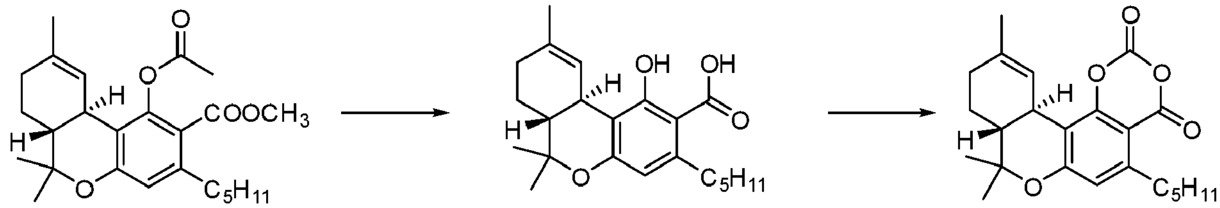

THC-acetate methyl ester (1.0 g, 2.4 mmol) was stirred for 24 h in a 1 M solution of LiOH in methanol (20 ml). The resulting solution was poured into 2 M aqueous HCl solution (100 ml) and extracted with dichloromethane (2 x 50 ml). The combined extracts were washed with saturated aqueous NaCl solution, dried over Na2SO4, filtered and concentrated to afford crude THCA. The crude THCA was dissolved in toluene (20 ml), trimethylamine (0.4 g, 4.0 mmol) and 2 M phosgene in toluene (2 ml, 4 mmol) was added. The mixture was stirred at room temperature until all THC was consumed, washed with saturated aqueous $NaHCO_3$ solution and dried over Na2SO4. Filtration and concentration of the filtrate afforded THCA carbonate (0.77 g, 83%).

Compound identity was confirmed by high-resolution mass spectrometry (HRMS): theoretical $C_{23}H_{28}O_5$ [M+Na]+ m/z = 407.18344; found: m/z = 407.18350.

The optical rotation was measured as $[a]_D^{20}$ = -50.1° (c = 1 g / 100 ml; in $CHCl_3$).

$^1$H-NMR spectra and $^{13}$C-NMR spectra were recorded and spectral data were extracted. Significant NMR signals are provided. The obtained THCA carbonate displays insufficient solubility in $CDCl_3$ and decomposition in d6-DMSO.

$^1$H-NMR ($CDCl_3$) significant peaks: δ=0.91 (t, 3 H, J=7.0 Hz, H-5"), 1.20-1.45 (m, 4 H, H-3",4"), 1.55 (s, 3 H, H-9'), 1.65 (s, 3 H, H-7'), 5.37 (s, 1 H, H-2').

$^{13}$C-NMR ($CDCl_3$) significant signals: δ=14.1 (C-5"), 22.3 (C-7'), 25.1 (C-9'), 124.0 (C-2'), 135.4 (C-3'), 146.5, 149.4 (C=O).

## Compounds and abbreviations

CBD; Cannabidiol; 2-[(1R,6R)-3-methyl-6-prop-1-en-2-ylcyclohex-2-en-1-yl]-5-pentylbenzene-1,3-diol.

CBDA; Cannabidiolic acid; 2,4-dihydroxy-3-[(1R,6R)-3-methyl-6-prop-1-en-2-ylcyclohex-2-en-1-yl]-6-pentylbenzoic acid.

THC; delta-9 Tetrahydrocannabinol; (6aR,10aR)-6,6,9-trimethyl-3-pentyl-6a,7,8,10a-tetrahydro-benzo[c]chromen-1-ol.

THCA; delta(9)-Tetrahydrocannabinolic acid; (6aR,10aR)-1-hydroxy-6,6,9-trimethyl-3-pentyl-6a,7,8,10a-tetrahydrobenzo[c]chromene-2-carboxylic acid.

THCV; Tetrahydrocannabivarin; (6aR, 1 0aR)-6,6,9-trimethyl-3-propyl-6a,7,8, 1 0a-tetrahydro-benzo[c]chromen-1-ol.

THCVA; Tetrahydrocannabivarinic acid; (6aR,10aR)-1-hydroxy-6,6,9-trimethyl-3-propyl-6a,7,8,10a-tetrahydrobenzo[c]chromen-2-carboxylic acid.

THCP; Tetrahydrocannabiphorol; (6aR,10aR)-6,6,9-trimethyl-3-heptyl-6a,7,8,10a-tetrahydro-benzo[c]chromen-1-ol.

THCPA; delta(9)-Tetrahydrocannabiphorolic acid; (6aR,10aR)-1-hydroxy-6,6,9-trimethyl-3-heptyl-6a,7,8,10a-tetrahydrobenzo[c]chromene-2-carboxylic acid.

## Claims

1. A compound according to Formula I

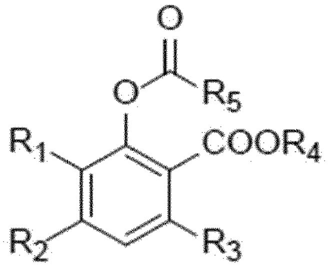

I

wherein $R^1$ = methyl-4-(prop-1-en-2-yl)-cyclohex-1-ene-3-yl (limonenyl);
wherein $R^2$ = acetate, propionate, butyrate, or OH;
wherein $R^3$ = propyl, pentyl, or heptyl;
wherein $R^4$ = methyl, ethyl, or propyl;
wherein $R^5$ = methyl, ethyl, or propyl,
or wherein $R^1$ = methyl-4-(2-propyl)-cyclohex-1-ene-3-yl or methyl-4-(2-propyl)-cyclohex-5-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ = methyl, ethyl, or propyl, and wherein $R^5$ = methyl, ethyl, or propyl,
or wherein $R^1$ = methyl-4-(2-propyl)-cyclohex-1-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ and $R^5$ vanish, i.e. are radicals forming a direct bond to each other.

**2.** Compound according to claim 1 having a Formula I

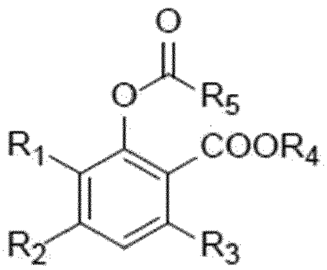

I

wherein $R^1$ = methyl-4-(prop-1-en-2-yl)-cyclohex-1-ene-3-yl (limonenyl);
wherein $R^2$ = acetate, or OH;
wherein $R^3$ = propyl, pentyl, or heptyl;
wherein $R^4$ = methyl;
wherein $R^5$ = methyl,
or wherein $R^1$ = methyl-4-(2-propyl)-cyclohex-1-ene-3-yl or methyl-4-(2-propyl)-cyclohex-5-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ = methyl, and wherein $R^5$ = methyl,
or wherein $R^1$ = methyl-4-(2-propyl)-cyclohex-1-ene-3-yl, wherein $R^2$ = O, and $R^1$ and $R^2$ together form a ring structure in which $R^2$ is an internal ring atom, wherein $R^3$ = propyl, pentyl, or heptyl, wherein $R^4$ and R5 vanish, i.e. are radicals forming a direct bond to each other.

**3.** Compound according to claim 1 or claim 2, selected from

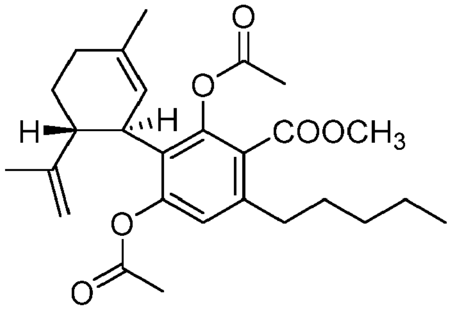

**II** (CBDA diacetate methyl ester),

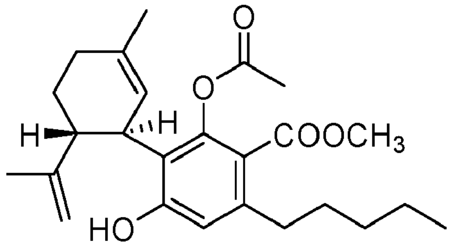

**III** (CBDA-2 acetate methyl ester),

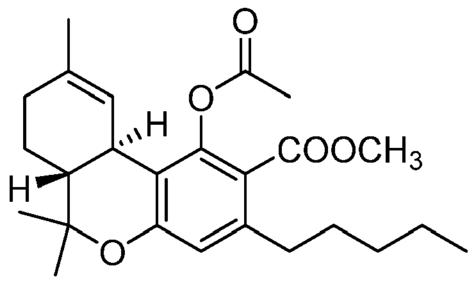

**IVa** (Delta-9-THCA acetate methyl ester),

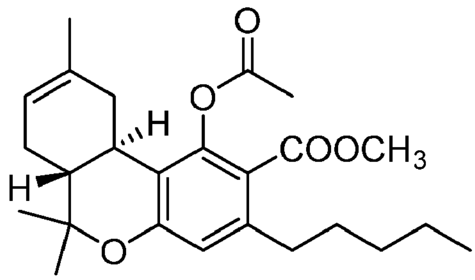

**IVb** (Delta-8-THCA acetate methyl ester), and

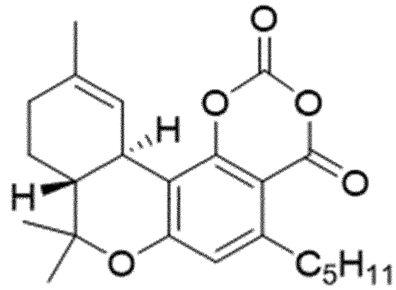

V (THCA carbonate).

4. Process for the synthesis and/or purification of tetrahydrocannabinolic acid (THCA) alkanoate and/or cannabidiolic acid (CBDA) di-alkanoate, preferably tetrahydrocannabinolic acid (THCA) acetate and/or cannabidiolic acid (CBDA) di-acetate, comprising the steps of

- providing a crude plant extract containing a mixture of cannabinoic acids, or providing CBDA;
- contacting and/or reacting the crude plant extract or the CBDA with an acylation agent, preferably an acetylation agent, and a chemical base, to obtain THCA alkanoate and/or CBDA di-alkanoate, preferably THCA acetate and/or CBDA di-acetate; and
- isolating the obtained THCA alkanoate and/or CBDA di-alkanoate, preferably THCA acetate and/or CBDA di-acetate,

wherein the acylation agent is selected from the list of $C_1$-$C_{10}$ aliphatic or aromatic anhydrides, preferably acetic anhydride,
wherein the chemical base is preferably a tertiary amine, such as pyridine or preferably tri-ethylamine,
wherein isolation is carried out by means of crystallization.

5. Process according to claim 4, wherein crystallization is performed in n-pentane or n-hexane.

6. Process according to claim 4 or claim 5, wherein the crude plant extract is provided by enzymatic extraction.

7. Process according to any one of claims 4 to 6, comprising the further step of treating the obtained CBDA di-acetate with an alkylation agent, such as e.g. $C_1$-$C_{10}$ dialkylsulfate, preferably dimethyl sulfate, to obtain a first CBDA di-acetate derivative, preferably CBDA diacetate methyl ester according to Formula II

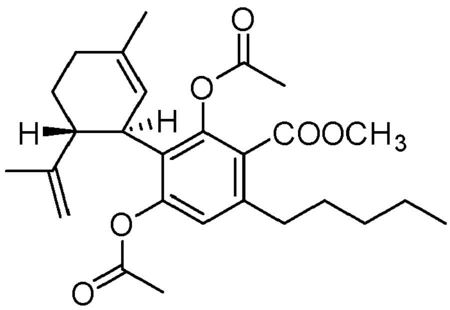

II.

8. Process according to claim 7, comprising the further step of deacylating the first CBDA di-acetate derivative to obtain a CBDA mono-acetate derivative, preferably CBDA-2-acetate methyl ester according to Formula III

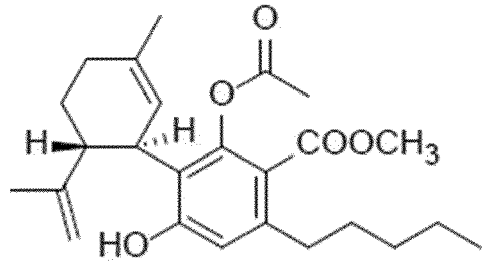

**III**.

9. Process according to claim 8, comprising the further step of converting the CBDA mono-acetate derivative into a THCA mono-acetate derivative, preferably THCA 2-acetate methyl ester according to Formula **IVa**

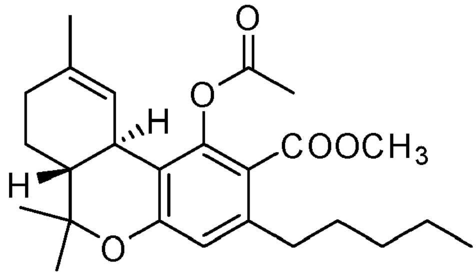

**IVa**.

10. Process according to claim 9, comprising the further step of converting THCA 2-acetate methyl ester, preferably via a THCA intermediate, into THCA carbonate according to Formula **V**

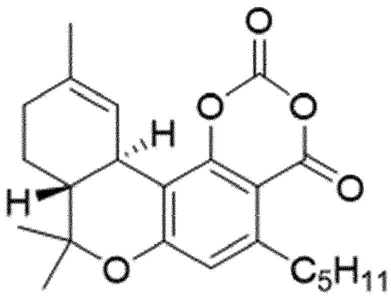

**V**.

11. Process according to any one of claims 7 to 10, wherein the obtained derivatives are at least 70% pure, preferably 90% pure, and more preferably 99% pure.

12. Process according to any one of claims 4 to 11, wherein a distillation step is absent and/or wherein a chromatography step is absent.

13. Process according to any one of claims 4 to 12, wherein the plant extract is obtained from a plant of the species *Cannabinaceae,* preferably a plant selected from the list of *Cannabis sativa*, *Cannabis indica*, and *Cannabis ruderalis*.

14. Compound according to any one of claims 1 to 3 for use in medicine.

15. Compound according to claim 14 for use in treating or preventing a disease associated with a cannabinoid receptor

in a subject in need thereof, wherein the cannabinoid receptor is one or more of CB1, CB2, 5HT1A, 5HT2A, GPR18, GPR55, GPRI 19, TRPVI, TPRV2, PPARy or a μ-opioid receptor.

Europäisches Patentamt
European Patent Office
Office européen des brevets

# EUROPEAN SEARCH REPORT

Application Number
EP 21 21 1470

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/298399 A1 (PEET RICHARD C [US] ET AL) 19 October 2017 (2017-10-19)<br>* schemes 1 and 2;<br>claim 1 *<br>----- | 1-3,14, 15 | INV.<br>C07C67/00<br>C07C67/31<br>C07C69/86<br>C07C69/94<br>C07D311/80<br>C07D493/04 |
| X | MECHOULAM R ET AL: "HASHISH-IV THE ISOLATION AND STRUCTURE OF CANNABINOLIC CANNABIDIOLIC AND CANNABIGEROLIC ACIDS", TETRAHEDRON, ELSEVIER SIENCE PUBLISHERS, AMSTERDAM, NL,<br>vol. 21, 1 January 1965 (1965-01-01), pages 1223-1229, XP009008207,<br>ISSN: 0040-4020, DOI: 10.1016/0040-4020(65)80064-3<br>* page 1225, first paragraph *<br>----- | 4-13 | |
| A | US 10 569 189 B1 (PAL KRUPAL DEVENDRA [CA] ET AL) 25 February 2020 (2020-02-25)<br>* column 2, lines 17-19, 43-53; claim 1 *<br>----- | 1-15 | |
| | | | TECHNICAL FIELDS SEARCHED (IPC) |
| | | | C07C<br>C07D |

The present search report has been drawn up for all claims

2

| Place of search | Date of completion of the search | Examiner |
|---|---|---|
| The Hague | 19 May 2022 | Matés Valdivielso, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 21 1470

This annex lists the patent family members relating to the patent documents cited in the above-mentioned European search report. The members are as contained in the European Patent Office EDP file on
The European Patent Office is in no way liable for these particulars which are merely given for the purpose of information.

19-05-2022

| Patent document cited in search report | Publication date | Patent family member(s) | Publication date |
|---|---|---|---|
| US 2017298399 A1 | 19-10-2017 | AU 2017250303 A1 | 15-11-2018 |
| | | CA 3021139 A1 | 19-10-2017 |
| | | CN 109311838 A | 05-02-2019 |
| | | EP 3442953 A1 | 20-02-2019 |
| | | JP 2019513422 A | 30-05-2019 |
| | | US 2017298399 A1 | 19-10-2017 |
| | | US 2019382814 A1 | 19-12-2019 |
| | | WO 2017181118 A1 | 19-10-2017 |
| US 10569189 B1 | 25-02-2020 | AU 2020200438 B1 | 25-06-2020 |
| | | CA 3067195 A1 | 26-03-2020 |
| | | CA 3081302 A1 | 26-03-2020 |
| | | DK 3738957 T3 | 20-09-2021 |
| | | EP 3738957 A1 | 18-11-2020 |
| | | KR 20200135299 A | 02-12-2020 |
| | | MA 51491 A | 24-03-2021 |
| | | NZ 761029 A | 27-11-2020 |
| | | US 10569189 B1 | 25-02-2020 |
| | | US 10792584 B1 | 06-10-2020 |
| | | US 2020398183 A1 | 24-12-2020 |
| | | US 2021379507 A1 | 09-12-2021 |
| | | WO 2020232526 A1 | 26-11-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **FORMATO et al.** *Molecules,* 2020, vol. 25, 2638
- **R. MECHOULAM ; Z. BEN-ZVI.** *Chem. Commun.,* 1969, 343-344